# EUROPEAN PATENT SPECIFICATION

(11) **EP 1 870 117 B1**
(45) Date of publication and mention of the grant of the patent: **10.11.2010**
(21) Application number: 07011661.1
(22) Date of filing: 14.06.2007
(51) Int. Cl.: A61L 27/30, A61L 27/34, A61L 29/08, A61L 29/10

(54) **Medical appliance coating composition and medical appliance**
Beschichtungszusammensetzung für medizinische Vorrichtungen und medizinische Vorrichtung
Composition de revêtement d'application médicale et appareil médical

(30) Priority: 21.06.2006 JP 2006171955
(43) Date of publication of application: 26.12.2007
(73) Proprietor: TERUMO KABUSHIKI KAISHA, Tokyo (JP)
(72) Inventor: Ishii, Shingo, c/o Terumo Kabushiki Kaisha, Fujinomiya-shi, Shizuoka-ken (JP); Onishi, Makoto, c/o Terumo Kabushiki Kaisha, Fujinomiya-shi, Shizuoka-ken (JP)
(74) Representative: Casalonga, Axel

(56) References cited:
- EP-A- 1 317 937
- WO-A-03/013199
- JP-A- 2005 168 855

## Description

The present invention relates to a medical appliance-coating composition capable of imparting a stable slidableness to a medical appliance. The present invention also relates to provide a medical appliance on which a slidable coating film having a stable slidableness is formed.

It is necessary to apply silicone oil or the like such as polysiloxane to a sliding portion or the like of a gasket for use in a pre-filled syringe as a lubricant to enhance the slidableness of the gasket and securely discharge a liquid medicine from the pre-filled syringe. But in dependence on a liquid medicine, it is difficult to pre-fill the liquid medicine in a syringe owing to an interaction of the liquid medicine with the lubricant For example, there is a case in which monoammonium glycyrrhetate which is an injection contacts the silicone oil to generate a foreign matter which is insoluble in water.

The method of applying the lubricant to the sliding portion of the gasket to enhance the slidableness of the gasket is widely used in disposable injection syringes. But it is desired that the lubricant is not applied to the sliding portion or the like of the gasket for use in the pre-filled syringe accommodating a medicine for hours in consideration of influence of the lubricant on the medicine accommodated therein.

The gasket for solving the above-described problem is disclosed in Japanese Patent Publication No.2001-104480. The gasket for solving the above-described problem is proposed by the present applicant as disclosed in USP 7,111,848. In this gasket, at least the periphery of the gasket body is covered with the film formed of fluororesin having a lower coefficient of friction than the gasket body. Supposing that the surface roughness of the resin film on the outer tube side thereof is defined as Ra₁ and that the surface roughness of the resin film on the gasket body side thereof is defined as Ra₂, the relationship of 1.5Ra₁ ≦ Ra₂ is satisfied. That the surface roughness Ra₁ is preferably in the range of 0.1 to 1.5µm is described in the specification.

In the medical appliance disclosed in Japanese Patent Publication No.2005-168855, (A) the inner surface and/or the surface of the base material to be treated is coated with the polymeric material containing carbon nanotube or (B) the thin film formed of the polymeric material containing the carbon nanotube is bonded to the inner surface and/or the surface of the base material to be treated.

The gasket disclosed in Japanese Patent Publication No.2001-104480 and in USP 7,111,848 works on a sufficiently high effect in dependence on a use condition. But there is a growing demand for the development of a gasket which has a basic function of preventing leak of a liquid when the gasket is subjected to a strict condition, for example, when an injection liquid containing a surface active agent is accommodated in the pre-filled syringe and when a medicine is administered under a high pressure. In addition this gasket is demanded to have a high degree of slidableness without using the lubricant such as the silicone oil.

There is a growing demand for the development of a medical appliance which has a high degree of slidableness and very accurate and stable medicine-discharge performance. When a liquid medicine is discharged from a pre-filled syringe by operating a syringe pump at a speed so low (for example, moving speed is about 2mm/hour when liquid medicine is discharged at 1mL/hour from syringe having diameter of 24mm) that the movement of the pump is unvisible, there may occur a discharge state called pulsation. In this case, there is a fear that the liquid medicine is prevented from being accurately administered.

The thin film disclosed in Japanese Patent Publication No.2005-168855 is effective to some extent in that the thin film is biocompatible, flexible, and resistant to kink, but is unpreferable in its slidableness.

The present invention has been made to solve the above-described problem. Therefore it is an object of the present invention to provide a medical appliance-coating composition capable of imparting a stable slidableness to a medical appliance without imparting a lubricant to the surface thereof. It is another object of the present invention to provide a medical appliance on which a slidable coating film having a stable slidable surface is formed.

The object described above is attained by the following a medical appliance-coating composition.

A medical appliance-coating composition, for imparting slidableness to said medical appliance, which contains a solvent-based silicone rubber, carbon nanotubes, and fine particles of silicone resin.

A medical appliance, having a slidable coating film formed thereon, which moves in contact with an inner surface of a medical member or an inner surface of a body lumen, wherein said slidable coating film is disposed at a portion, of said medical appliance, which contacts said medical member or said body lumen; and said slidable coating film is comprised of silicone rubber, carbon nanotubes and fine particles of silicone resin.
Fig.1 is a front view showing a gasket for use in a syringe, of an embodiment of a medical appliance of the present invention.
Fig. 2 is a sectional view showing the gasket shown in Fig.1.
Fig. 3 is a top view showing the gasket shown in Fig.1.
Fig. 4 is a bottom view showing the gasket shown in Fig.1.
Fig. 5 is a sectional view showing a pre-filled syringe of an embodiment of the present invention.
Fig. 6 is a sectional view showing a guide wire of an embodiment of the medical appliance of the present invention.
Fig. 7 is a table 1a showing components of medical appliance-coating compositions of examples of the present invention and compounding ratio thereof.
Fig. 8 is a table 1b showing components of medical appliance-coating compositions of comparison examples and compounding ratio thereof.
Fig. 9 shows the surface of a slidable coating film which is formed by applying a medical appliance-coating composition of an example 1 to a substrate and is photographed by an electron microscope.
Fig. 10 shows the surface of a guide wire of an example 18 photographed by an electron microscope.

The medical appliance-coating composition (hereinafter often referred to as merely composition or slidable composition) of the present invention and the medical appliance of the present invention having a slidable coating film formed thereon will be described below. The medical appliance-coating composition of the present invention is a medical appliance-coating slidable composition

Initially the composition of the present invention is described below.

The composition of the present invention imparts slidableness to the medical appliance and contains solvent-based silicone rubber and in addition, carbon nanotubes and fine particles of silicone resin.

The composition of the present invention contains solvent-based silicone rubber as its main component and in addition, carbon nanotubes and fine particles of silicone resin.

A liquid organic polysiloxane compound is used as the solvent-based silicone rubber used for the composition of the present invention.

The organic polysiloxane compound which is used in the present invention is a polymer containing a repeated structural unit of an organic silicone compound indicated by R1(R2)-Si-O in the backbone thereof. R1 and R2 denote lower alkyl groups having 1 to 8 carbons. As the organic polysiloxane compound, it is possible to use polyalkylsiloxanes (more specifically, polydimethylsiloxane, polyethylsiloxane, polymethylphenylsiloxane, and polydiphenylsiloxane), polyalkyl hydrogen siloxane, and substances having epoxy group, hydroxyl group, carboxylic group, vinyl group, amino group, alkoxy group or fluorine group introduced into ends or molecular chains of the above-described polymers.

As the organic polysiloxane compound, it is possible to use both homopolymer-type organic polysiloxane composed of one kind of a repeated structural unit and random-type, block-type or graft-type organic polysiloxane composed of a combination of not less than two kinds of repeated structural units.

As the organic silicone compound constituting the repeated structural unit, it is possible to list methyltrimethoxysilane, methyltriethoxysilane, methyltriisopropoxysilane, methyltri-t-butoxysilane, methyltrichlorosilane, methyltribromosilane; ethyltrimethoxysilane, ethyltriethoxysilane, ethyltriisopropoxysilane, ethyltri-t-butoxysilane, ethytrichlorosilane, ethyltribromosilane; n-propyltrimethoxysilane, n-propyltriethoxysilane, n-propyltriisopropoxysilane, n-propyltri-t-butoxysilane, n-propyltrichlorosilane, n-propyltribromosilane; n-hexyltrimethoxysilane, n-hexyltriethoxysilane, n-hexyltriisopropoxysilane, n-hexyltri-t-butoxysilane, n-hexyltrichlorosilane, n-hexyltribromosilane; n-decyltrimethoxysilane, n-decyltriethoxysilane, n-decyltriisopropoxysilane, n-decyltri-t-butoxysilane, n-decyltrichlorosilane, n-decyltribromosilane; n-octadecyltrimethoxysilane, n-octadecyltriethoxysilane, n-octadecyltriisopropoxysilane, n-octadecyltri-t-butoxysilane, n-octadecyltrichlorosilane, n-octadecyltribromosilane, tetramethoxysilane, tetraethoxysilane, tetrabutoxysilane, dimethoxydiethoxysilane; dimethyldimethoxysilane, dimethyldiethoxysilane, γ-glycydoxypropylmethyldimethoxysilane, γ-glycydoxypropylmethyldiethoxysilane, γ -glycydoxypropyltrimethoxysilane, γ -glycydoxypropyltriethoxysilane, γ -glycydoxypropyltriisopropoxysilane, and γ -glycydoxypropyltri-t-butoxysilane.

An organic solvent is used for the composition of the present invention. Toluene, xylene, and the like are used as the organic solvent Although the content of the solvent-based silicone rubber is different according to the content of a nonvolatile content thereof, it is preferable that the solvent-based silicone rubber is contained at 5 to 40 wt% in the composition containing the organic solvent The content of the organic polysiloxane compound which is the nonvolatile content of the solvent-based silicone rubber contained in the medical appliance-coating composition containing the organic solvent is set favorably to the range of 0.5 to 20 wt% and more favorably to the range of 10 to 70 wt%.

Carbon nanotubes are contained in the composition of the present invention.

The carbon nanotube is cylindrically formed of a network (graphene sheet) of six-membered rings made of carbon. The carbon nanotube is classified into a single-layer carbon nanotube and a multi-layer carbon nanotube.

In the present invention, it is possible to use the single-layer carbon nanotube or/and the multi-layer carbon nanotube. The carbon nanotube which is used in the present invention is the multi-layer carbon nanotube having an average diameter less than 800nm and preferably 10 to 250nm and an average fiber length less than 2mm and preferably in the range of 0.001 to 1mm. The carbon nanotube is contained in the composition in the range of 0.01 to 2 wt% and preferably in the range of 0.01 to 1 wt%. The content of the carbon nanotube contained in the composition is favorably 1/3000 to 1/16 and more favorably 1/1000 to 1/30 of the content of the organic polysiloxane compound which is the nonvolatile content of the solvent-based silicone rubber contained in the composition.

The slidable composition of the present invention contains fine particles of silicone resin. The size of the fine particle of the silicone resin is in the range of 0.5 to 30µm, favorably in the range of 1 to 10µm, and more favorably in the range of 2 to 8µm. The fine particle of the silicone resin is adhesive to the component of the solvent-based silicone rubber. Fine particles of the silicone resin called organopolysiloxane containing dimethylpolysiloxane therein as its main component is preferable. The fine particle of the silicone resin may contain functional groups such as a vinyl group, an epoxy group, an amino group, and the like. The fine particles of the silicone resin having a structure in which a siloxane bond is crosslinked in a three-dimensional network, namely, the structure of polymethyl silsesquioxane is especially favorable. The silicone resin may consist of fine particles of one kind thereof or in combination of two or more kinds thereof.

It is preferable that the fine particles of the silicone resin are contained at 0.2 to 50 wt% and favorably 1 to 20 wt% in the composition. The fine particles of the silicone resin are contained in the composition at favorably 1/150 to 1/1 and more favorably 1/30 to 1/1.5 of the content of the organic polysiloxane compound which is the nonvolatile content of the solvent-based silicone rubber contained in the composition.

It is preferable that the composition of the present invention contains a surface active agent (in other words, surfactant).

As the surface active agent, a nonionic surface active agent is preferable. As the nonionic surface active agent, it is possible to use polyoxyethylenealkylphenyl ethers having alkyl groups such as octyl, nonyl, dodecyl; polyoxyethylenealkyl ethers having alkyl groups such as octyl, lauryl, cetyl, stearyl, and oleyl; polyethylene glycol fatty esters such as monooleate, monostearate, distearate, monolaurate of polyethylene glycol; polyoxyethylenealkylamines having the alkyl group such as the lauryl, the stearyl, and the oleyl; sorbitan fatty esters such as sorbitan sesquioleate, sorbitan sesquiisostearate, sorbitan monolaurate, sorbitan monopalmitate, sorbitan monostearate, sorbitan distearate, sorbitan tristearate, sorbitan monooleate, sorbitan trioleate; polyoxyethylene sorbitan fatty esters such as polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, polyoxyethylene sorbitan trioleate, and polyoxyethylene sorbitan of fatty ester of coconut oil; polyethylene glycol ethers, fatty diethanolamides such as diethanolamide laurate; sucrose fatty esters; fatty monoglycerides such as monoglyceride laurate, monoglyceride stearate, monoglyceride oleate; polyoxyethylene fatty amides such as polyoxyethylene lauramide, polyoxyethylene stearamide, polyoxyethylene oleamide; and polyoxyethylene castor oil, polyoxyethylene hardened castor oil.

The surface active agent is contained at 0.05 to 5 wt% and preferably 0.5 to 3 wt% in the composition. The surface active agent is contained in the composition at favorably 1/600 to 1/6 and more favorably 1/60 to 1/10 of the content of the organic polysiloxane compound which is the nonvolatile content of the solvent-based silicone rubber contained in the composition.

It is preferable that the composition of the present invention contains an adhesion-improving agent A silane coupling agent is preferable as the adhesion-improving agent As the silane coupling agent it is possible to use an acrylic (methacrylic) functional silane coupling agent, an epoxy functional silane coupling agent, and an amino (imino) functional silane coupling agent

As the acrylic (methacrylic) functional silane coupling agent, it is possible to list 3-methacryloxypropyl trimethoxy silane, 3-methacryloxypropyl triethoxy silane, 3-acryloxypropyl trimethoxy silane, 3-acryloxypropyl triethoxy silane, methacryloxymethyl trimethoxy silane, methacryloxymethyl triethoxy silane, acryloxymethyl trimethoxy silane, and acryloxymethyl triethoxy silane.

As the epoxy functional silane coupling agent, it is possible to list 3-glycidoxypropyltrimethoxysilane, 3-glycidoxypropyltriethoxysilane, 2-(3,4-epoxycyclohexyl)ethyltrimethoxysilane, and 2-(3,4-epoxycyclohexyl)ethyltriethoxysilane.

As examples of the amino (imino) functional silane coupling agent it is possible to list alkoxysilane, containing an amino group and (or) an imino group, such as H₂NCH₂ CH₂CH₂Si(OCH₃)₃, H₂NCH₂CH₂NHCH₂CH₂CH₂Si(OCH₃)₃, H₂NCH₂CH₂NHCH₂CH₂CH₂Si(CH₃)(OCH₃)₂, and (C₂H₅O)₃Si(CH₂)₃NH(CH₂)₂NH(CH₂)₃Si(OC₂H₅)₃ and products formed by reaction between the alkoxysilane containing the amino group and (or) the imino group and methacryloxy silane compounds such as CH₂=C(CH₃)COOCH₂CH₂CH₂Si(OCH₃)₃, and CH₂=C(CH₃)COOCH₂CH₂CH₂Si(OCH₂CH₂OCH₃)₃.

The silane coupling agent is contained in the composition favorably at 1/100 to 1/5 and more favorably at 3/100 to 1/10 of the content of the organic polysiloxane compound which is the nonvolatile content of the solvent-based silicone rubber contained in the composition.

It is preferable that the composition of the present invention contains powder of the resin of the polyolefin family.

As the fine particle of the resin of the polyolefin family, fine particles of polyethylene, polypropylene, and the like can be used. Fine particles of only one kind of the above-described synthetic resins or mixtures thereof may be used.

The size of the fine particle of the resin of the polyolefin family is set to the range of 0.5 to 10µm and favorably to the range of 2 to 8µm.

The fine particles of the resin of the polyolefin family are contained in the composition at 0.1 to 50 wt% and favorably at 1 to 20 wt%. The fine particles of the resin of the polyolefin family is contained in the composition at favorably 1/150 to 1/1 and more favorably 1/30 to 1/1.5 of the content of the organic polysiloxane compound which is the nonvolatile content of the solvent-based silicone rubber contained in the composition.

It is preferable that the composition of the present invention contains powder of mica.

As the powder of the mica, natural mica powder or synthetic mica powder can be used. The synthetic mica powder is classified into an unswelling-type mica (for example, fluorine phlogopite, potassium tetrasilisic mica) and swelling-type mica (for example, sodium tetrasilisic mica, sodium hectorite). Although both can be used, the unswelling-type mica is favorable.

The size of the mica powder is set to the range of 0.5 to 10µm and favorably the range of 2 to 8µm.

The powder of the mica is contained in the composition at 0.1 to 50 wt% and favorably at 1 to 15 wt%. The powder of the mica is contained in the composition at favorably 1/300 to 1/1.5 and more favorably 1/30 to 1/2 of the content of the organic polysiloxane compound which is the nonvolatile content of the solvent-based silicone rubber contained in the composition.

The composition of the present invention may contain a catalyst for obtaining silicone rubber of condensation type by means of a crosslinking reaction. The catalyst may be added to the solvent-based silicone rubber when the composition is used. For example, as the catalyst, it is possible to use organic carboxylate or the ester of metals such as titanium, tin, zinc, cobalt, lead, calcium, manganese. More specifically, it is possible to use dialkyl tin compounds, titanium dipropoxide bisacetylacetonate, dibutyl tin laurate, cobalt naphthenate, and platinic chloride.

The composition of the present invention is prepared as follows:

Initially a surface active agent is added to a solvent (for example, organic solvent). Carbon nanotubes are added to the solvent to which the surface active agent has been added. The solution is stirred to prepare a liquid in which the carbon nanotubes are preferably dispersed. Thereafter the solvent-based silicone rubber is added to the liquid in which the carbon nanotubes have been dispersed. Thereby the composition is prepared. Therefore in the composition, the solvent-based silicone rubber is dispersed or dissolved in the solvent in which the carbon nanotubes are dispersed owing to the presence of the added surface active agent

Thereafter the adhesion-improving agent and the catalyst are used as necessary.

It is possible to use the composition of the present invention of a two component type. In this case, to form the composition, the surface active agent, the carbon nanotube, the solvent-based silicone rubber, and the adhesion-improving agent which is used as necessary are added to the organic solvent to from the main agent whereas the auxiliary agent consisting of the catalyst is added to the main agent at the time of use. Alternatively it is possible to compose the main agent of a mixture of the organic solvent, the surface active agent, the carbon nanotube, and the solvent-based silicone rubber and compose the auxiliary agent of the adhesion-improving agent and the catalyst both of which are added to the main agent at the time of use.

The details of the solvent the surface active agent the carbon nanotube, the solvent-based silicone rubber, the adhesion-improving agent and the catalyst are as described above. The addition amount of each of these components is as described above.

The coefficient of dynamic friction of the slidable coating film made of the composition of the present invention is set favorably to not more than 0.15 and more favorably to not more than 0.13, when the coefficient of dynamic friction thereof is measured in accordance with the method of JIS K 7125.

The medical appliance having the slidable coating film (in other words, slidable coating layer) of the present invention formed thereon is described below.

The medical appliance 1 of the present invention having the slidable coating film formed thereon moves in contact with an inner surface of a medical member or an inner surface of a body lumen (for example, blood vessel, alimentary canal, abdominal cavity) and has a slidable coating film 3 formed at a portion thereof which contacts the medical member or the body lumen. The slidable coating film 3 is comprised of silicone rubber, carbon nanotubes and fine particles of silicone resin.

In the medical appliance 1 of the present example, the slidable coating film 3 is composed of the silicone rubber and contains the carbon nanotubes and the fine particles of the silicone resin.

The slidable coating film 3 is formed as a rough surface. The rough surface is formed mainly of the fine particles of the silicone resin. The slidable coating film 3 is reinforced with the carbon nanotube. A part of the rough surface is formed of the carbon nanotube.

The medical appliance of the present invention having the slidable coating film formed thereon is described below by using an embodiment in which the medical appliance is used as a gasket for use in a syringe and incorporated in the syringe.

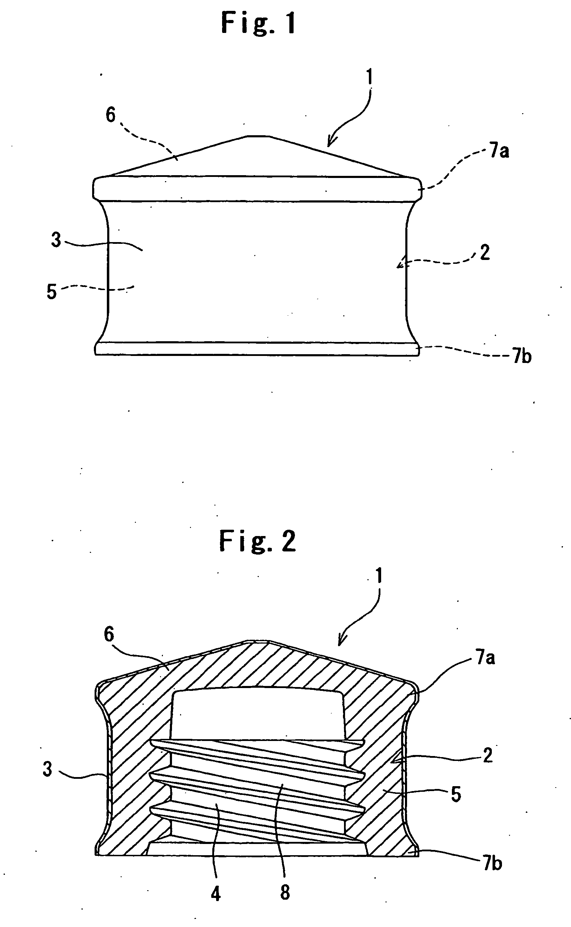

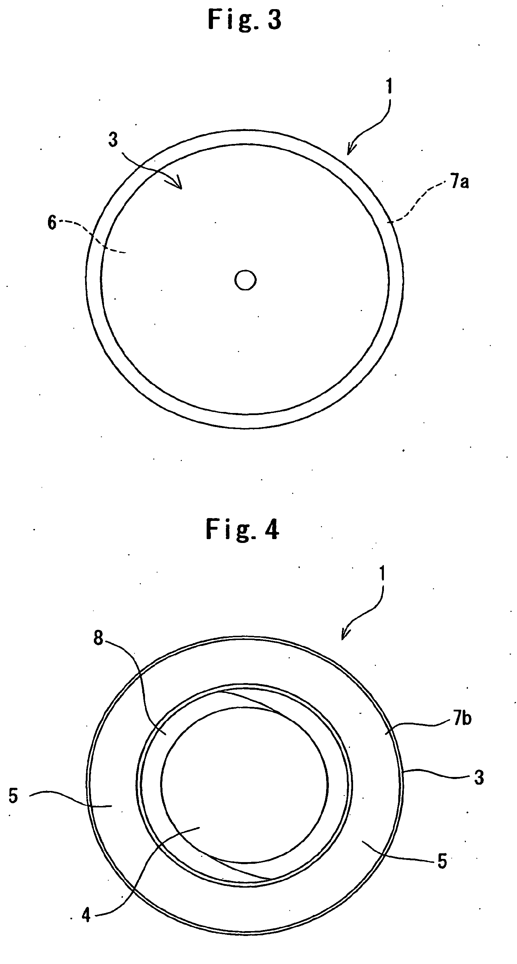

Fig.1 is a front view showing a gasket, for use in a syringe, of an embodiment of a medical appliance of the present invention. Fig. 2 is a sectional view showing the gasket shown in Fig. 1. Fig. 3 is a top view showing the gasket shown in Fig. 1. Fig. 4 is bottom view showing the gasket shown in Fig.1.

The medical appliance of this embodiment having the slidable coating film formed thereon is a gasket 1 for use in a syringe and accommodated liquid-tightly and slidably inside an outer cylinder 11, for use in the syringe, which is a medical member. The gasket 1 has the slidable coating film 3 disposed at a portion thereof which contacts the outer cylinder 11. The slidable coating film 3 has a rough surface attributed to the presence of the fine particle of the silicone resin contained therein. The gasket 1 has a core part 2 and the slidable coating film 3 formed on a portion, of an outer surface of the core part 2, which contacts an inner surface of the outer cylinder 11. The slidable coating film 3 may be formed on the entire outer surface of the core part 2.

A medical appliance of this embodiment is a syringe 10. The syringe 10 has a gasket 1, for use in the syringe, which is a first medical member; and an outer cylinder 11, for use in the syringe, which is a second medical member and accommodates the gasket 1 therein liquid-tightly and slidably. The slidable coating film 3 is provided at a portion, of the gasket 1, which contacts the outer cylinder 11. In forming the slidable coating film 3 on the gasket 1, the slidable coating film 3 is disposed on a portion, of the outer surface of the core part 2, which contacts the inner surface of the outer cylinder 11. The slidable coating film 3 may be formed on the entire outer surface of the core part 2. When the slidable coating film 3 is formed on the inner surface of the outer cylinder 11, the slidable coating film 3 is formed on the inner surface of a region, of the outer cylinder 11, in which the gasket 1 slidably moves.

The gasket and the syringe which are an embodiment of the medical appliance of the present invention are described below.

As shown in Figs. 1, 2, and 5, the core part 2 of the gasket 1 for use in the syringe has a main body 5 extending in an almost equal diameter; a tapered portion 6, disposed at a distal side of the main body 5, whose diameter decreases taperingly to the distal end thereof; a plunger-mounting portion 4 provided inside the main body 5 from a proximal end thereof toward a distal side thereof; a distal-side annular rib 7a provided on a side surface of the distal portion of the main body 5, and a proximal-side annular rib 7b provided on a side surface of the proximal portion of the main body 5.

As shown in Figs. 2 and 4, the plunger-mounting portion 4 is formed as a concave portion, approximately columnar, which is disposed inside the main body 5 and extends from the proximal end of the main body 5 to a position in the vicinity of the distal end thereof. A screwing portion 8 capable of engaging a screwing portion formed at a distal end of a plunger is formed on a side surface of the concave portion. A distal-end surface of the concave portion is formed almost flatly. The plunger-mounting portion 4 does not necessarily have to be formed as the screwing portion, but may be formed as an engaging portion which engages the distal portion of the plunger.

The outer diameters of the annular ribs 7a and 7b are formed a little larger than the inner diameter of the outer cylinder 11 for use in the syringe. Therefore the annular ribs 7a and 7b compressively deform inside the outer cylinder 11. In the embodiment, two annular ribs are formed, but three or more annular ribs may be formed.

It is preferable that an elastic material is used to compose the core part 2. As the elastic material, it is possible to list rubber materials (vulcanized rubber materials are preferable) such as natural rubber, isoprene rubber, butyl rubber, chloroprene rubber, nitrile-butadiene rubber, styrene-butadiene rubber, silicone rubber; styrene elastomer and hydrogenated styrene elastomer; and mixtures of the styrene elastomer and polyolefins such as polyethylene, polypropylene, polybutene, and α-olefin copolymers; mixtures of the styrene elastomer and oil such as liquid paraffin, process oil; and mixtures of the styrene elastomer and powdery inorganic substances such as talc, cast, mica, and the like. Further it is possible to use polyvinyl chloride elastomer, olefin elastomer, polyester elastomer, polyamide elastomer, polyurethane elastomer, and mixtures of these elastomers as the materials for the core part 2. As the elastic material, the diene rubbers and the styrene elastomer are especially preferable because these rubbers or elastomers are favorable in elastic properties and resistant to sterilization by steam.

It is essential that the slidable coating film 3 is formed at the annular ribs. More specifically, it is essential that the slidable coating film is formed at the distal-side annular rib 7a and the proximal-side annular rib 7b.

The thickness of the slidable coating film 3 is set favorably to the range of 1 to 20µm and more favorably to the range of 3 to 10µm. When the thickness of the slidable coating film 3 is not less than 1µm, the slidable coating film 3 works on a sufficiently high slidable performance. When the thickness of the slidable coating film 3 is not more than 10µm, the slidable coating film 3 can be securely formed as the rough surface attributed to the presence of the fine particle of the silicone resin contained therein without adversely affecting the elasticity of the gasket

The slidable coating film 3 is composed of the silicone rubber as its main component and contains the carbon nanotubes and the fine particles of the silicone resin. In other words, the slidable coating film 3 is composed of the coating film made of the silicone rubber, the carbon nanotubes and the fine particles of the silicone resin both held by the coating film. The coating film disposed on the surface of the slidable coating film is flexible. Further the slidable coating film is highly slidable owing to the rough surface thereof formed of the fine particles of the silicone resin contained therein. Furthermore, the carbon nanotubes contained in the slidable coating film impart a strength thereto to such an extent that the slidable coating film stands up a resistance force generated when the slidable coating film 3 slides in contact with the inner surface of the outer cylinder 11. The slidable coating film can be formed by applying the (slidable) composition on the core part of the gasket and hardening it

The coefficient of dynamic friction of the slidable coating film to be formed on the gasket is favorably not more than 0.15 and more favorably not more than 0.13, when the coefficient of dynamic friction thereof is measured in accordance with the method of JIS K 7125.

As the silicone rubber which is used as the main component of the slidable coating film 3, it is preferable to use the hardened solvent-based silicone rubber. A liquid organic polysiloxane compound is used as the solvent-based silicone rubber. Therefore the hardened liquid organic polysiloxane compound is preferable as the silicone rubber. The above-described organic polysiloxane compounds can be preferably used. More specifically, the hardened liquid organic polysiloxane compound is preferably the crosslinked organic polysiloxane compound. The Young's modulus of the silicone rubber is favorably not more than 3,000 MPa and more favorably not more than 1,000 MPa.

The slidable coating film 3 contains a large number of fine particles of the silicone resin held by the coating film of the silicone rubber. The slidable coating film 3 has the rough surface attributed to the presence of the fine particles of the silicone resin contained therein. Owing to the rough surface, the slidable coating film 3 has a stable slidableness.

The surface roughness (Ra, measuring method: JIS B 0601: 1994) of the slidable coating film 3 is favorably in the range of 1.7 to 4.5µm and more favorably in the range of 2.0 to 3.0µm. The surface roughness Ra1 described in USP 7,111,848 is sufficiently higher than the surface roughness of 0.1 to 1.5µm. The detail of the fine particle of the silicone resin is as described previously. The size of the fine particle of the silicone resin is set to the range of 0.5 to 10µm and favorably to the range of 2 to 8µm. The ratio of the average diameter of the fine particle of the silicone resin to the average film thickness (average film thickness including fine particles) of the slidable coating film 3 is set favorably to 0.2 to 1.5, more favorably to 0.3 to 1.2, and most favorably to 0.4 to 1.0. When the above-described ratio is below 1.5, the rough surface does not cause a decrease in the air-tightness of the slidable coating film 3. When the ratio is above 0.2, the surface of the slidable coating film 3 can be roughened to a sufficiently hight extent The fine particles of the silicone resin are adhesive to the coating film of the silicone rubber. The content of the fine particles of the silicone resin is 3 to 60% of the weight (weight after slidable coating film hardens) of the slidable coating film and 2 to 60% in a volume ratio. The above-described fine particles of the silicone resin are used in the present invention.

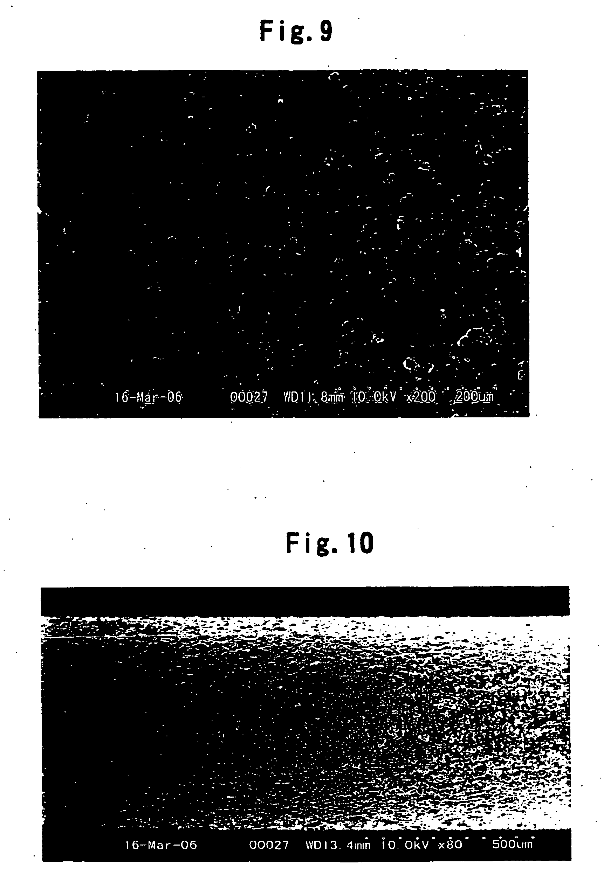

Because the gasket 1 of the present invention has the above-described slidable coating film 3 formed thereon, the gasket 1 has a stable slidableness without applying a lubricant to the sliding surface thereof and is capable of maintaining sealing performance of a space for accommodating a medicine. Because the slidable coating film 3 contains the fine particles, it has the rough surface as shown in Fig. 9.

The slidable coating film 3 contains a large number of the carbon nanotubes held by the coating film of the silicone rubber. The above-described carbon nanotubes are used. It is preferable that the content of the carbon nanotube is 0.01 to 3% of the weight (weight of fine particle after it hardens) of the slidable coating film and 0.004 to 5% thereof in a volume ratio.

It is preferable that the slidable coating film 3 contains the powder of the resin of the polyolefin family. The kind of the fine particles of the resin of the olefin family to be used in the present invention is previously described. It is preferable that the content of the powder of the resin of the polyolefin family is set to the range of 3 to 60% of the weight (weight of fine particle after it hardens) of the slidable coating film and to the range of 4 to 65% thereof in a volume ratio.

It is preferable that the slidable coating film 3 contains the powder of the mica. The kind of the mica to be used in the present invention is previously described. It is preferable that the content of the powder of the mica is set to the range of 1 to 40% of the weight (weight of fine particle after it hardens) of the slidable coating film and 0.4 to 15% thereof in a volume ratio.

The slidable coating film 3 may contain the surface active agent The kind of the surface active agents to be used in the present invention is previously described. It is preferable that the content of the surface active agent is set to the range of 0.05 to 5% of the weight of (weight of fine particle after it hardens) of the slidable coating film.

The slidable coating film 3 may contain the adhesion-improving agent The kind of the adhesion-improving agent to be used in the present invention is previously described. It is preferable that the content of the adhesion-improving agent is set to the range of 10 to 50% of the weight of (weight of fine particle after it hardens) the slidable coating film.

The slidable coating film 3 may contain the catalyst for obtaining the silicone rubber of a condensation type by means of a crosslinking reaction. The kind of the catalyst to be used in the present invention is previously described.

The method of forming the slidable coating film 3 is described below.

Initially the above-described composition is prepared.

The slidable coating film is obtained by applying the composition to the clean surface of the gasket and hardening it At this time, the composition can be applied to the surface of the gasket by carrying out a known method such as a dip coating method, a spraying method, and the like. It is preferable that a coating solution is sprayed to the surface of the object to be coated with the object being rotated (at 100 to 600 rpm). It is preferable to heat a portion of the gasket to be coated to 50 to 100°C before the coating solution is sprayed thereto. Thereby the composition can be rapidly fixed to the surface of the portion of the gasket to be coated. Therefore it is easy to carry out this method.

As the method of hardening the composition, it may be left at a normal temperature, but it is preferable to heat it The method of thermally hardening the composition is not limited to a specific method, provided that the base material of the gasket is not modified or deformed. It is possible to adopt conventional methods of drying the composition by hot air, drying the composition by an oven utilizing infrared rays or by a drier which is used under a reduced pressure. The composition can be hardened in a short period of time by using the coating solution containing the silane coupling agent In forming the slidable coating film on the gasket, the concentration of the mixed solution is appropriately controlled and the dipping method, the spraying method or the like is carried out

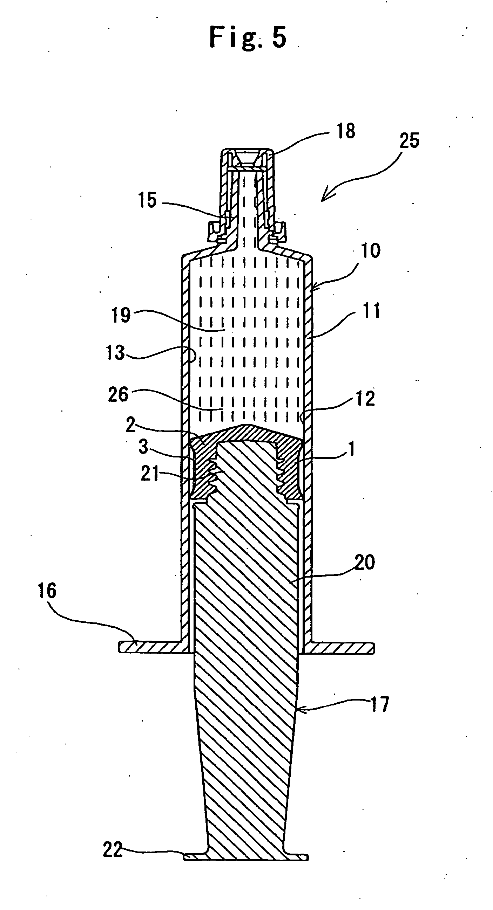

As shown in Fig. 5, the syringe 10 which is an embodiment of the medical appliance of the present invention includes an outer cylinder 11, for use in the syringe, which has a needle-mounting portion 15 disposed at a distal side thereof and a flange 16 disposed at a proximal end thereof; a gasket 1, for use in the syringe, which is capable of liquid-tightly and airtightly sliding on an inner surface 12 of the outer cylinder 11; a plunger 17 which is or can be mounted on the gasket 1; a sealing member 18 for sealing the needle-mounting portion 15 of the outer cylinder 11; and a medicine-accommodating portion 19, for accommodating a medicine 26, which is surround with the sealing member 18, the inner surface 12 of the outer cylinder 11 (and the inner surface of the slidable coating film 3), and the gasket 1. Instead of the sealing member 18, a needle may be mounted on the needle-mounting portion 15. As shown in Fig. 5, the sealing member 18 into which a double ended needle can be directly inserted is preferable.

As shown in Fig. 5, the above-described medical appliance is a pre-filled syringe 25 including the syringe 10 and the medicine 26.

The outer cylinder 11 for use in the syringe has the needle-mounting portion 15 disposed at the distal portion thereof and the flange 16 disposed at the proximal end thereof. The outer cylinder 11 is made of a material transparent or semitransparent It is preferable that the outer cylinder 11 is made of a material having low oxygen permeability or vapor permeability. It is preferable that the material of the outer cylinder 11 has glass transition point or a melting point not less than 110°C.

As the material to form the outer cylinder 11, the following resins are preferable: polyolefin such as polypropylene, polyethylene, poly (4-methylpentene-1), and cyclic polyolefin; polyesters such as polyethylene terephthalate, polyethylene naphthalate, and non-crystalline polyarylate; polystyrene; polyamide; polycarbonate, polyvinyl chloride; acrylic resin; acrylonitrile-butadiene-styrene copolymer, and non-crystalline polyetherimide. The polypropylene, the poly (4-methylpentene-1), the cyclic polyolefin, the polyethylene naphthalate, and the non-crystalline polyetherimide are especially preferable because these resins are transparent and resistant to steam sterilization under pressure. These resins can be used as a material to form not only the outer cylinder but also containers capable of accommodating medicines.

The gasket 1, for use in the syringe, having the slidable coating film 3 is used in the embodiment of the present invention. The construction of the gasket 1, for use in the syringe, having the slidable coating film 3 is as described above.

As shown in Fig. 5, the plunger 17 has a sectionally cross-shaped main body 20 extended axially; a plunger-side screwing portion 21, provided at a distal portion thereof, which engages the plunger-mounting portion 4; a disk portion 22, for pressing use, which is disposed at a proximal end of the main body 20; and a rib formed midway on the main body 20.

The medicine 26 is contained in the syringe 10 of this embodiment As the medicine 26, it is possible to use a liquid medicine insoluble in water, a liquid medicine having a high adsorbability, a liquid medicine containing a surface active agent and having a low viscosity and a high degree of penetration, and liquid medicines containing an electrolytic substance, vitamins, minerals, and antibiotics. It is also possible to use powdery medicine such as protein preparation, a freeze-drying agent A liquid drug is also used as the medicine 26.

As the material composing the plunger 17 and the sealing member 18, it is preferable to use hard resin or semi-hard resin such as high-density polyethylene, polypropylene, polystyrene, polyethylene terephthalate, and the like.

The above-described syringe is an example of medical appliances which move in contact with the inner surface of a medical member. The medical appliances of any types can be used, provided that they slidably contact the inner surface of the medical member. For example, the medical appliance may be a rubber stopper-provided vial, a transfusion bag, a blood collection tube, a decompression blood collection tube. The medical appliance of the present invention may be an O-ring, a plug, a lid, and the like, provided that these medical appliances slidably contact the medical member. For example, the medical appliance may be a rubber stopper of the vial, the lid of the transfusion bag, and the like.

The medical appliance of the present invention may move in contact with the inner surface of a body lumen. The medical appliance which moves in contact with the inner surface of the body lumen includes a catheter, a guide wire, a vasodilating device, and the like. The medical appliance of the present invention may move in contact with the inner surface of the medical member and that of the lumen. The medical appliance which moves in contact with the inner surface of the lumen includes the catheter which is inserted into another catheter (for example, guiding catheter), the guide wire, the vasodilating device, and the like, the distal ends of which are guided to a desired portion of the lumen.

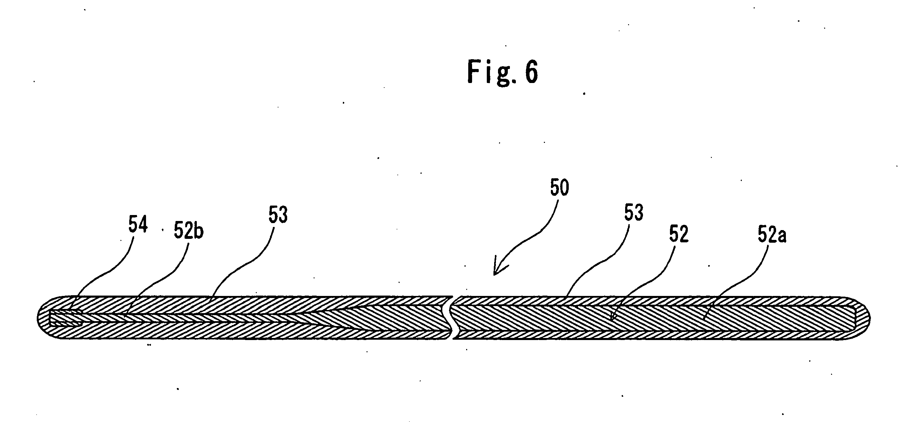

An embodiment in which the medical appliance of the present invention is applied to the guide wire is described below with reference to Fig. 6.

Fig. 6 is a sectional view an embodiment of the guide wire of the present invention.

A guide wire 50 of this embodiment has an inner core 52 and a slidable coating film 53 covering the inner core 52. The slidable coating film 53 is comprised of the silicone rubber, the carbon nanotube, and the fine particle of the silicone resin. In this embodiment, the slidable coating film 53 is composed of silicone rubber, and contains carbon nanotubes and fine particles of silicone resin.

The guide wire of the embodiment shown in Fig. 6 has the inner core 52 including a main body 52a having a higher rigidity and a distal portion 52b, formed integrally with the main body 52a, which has a smaller diameter than the main body 52a and a lower rigidity; a high X-ray opaque portion 54 disposed at a distal end of the inner core 52; and a slidable coating film 53 coating the entire inner core 52 having the high X-ray opaque portion 54 provided at the distal end thereof. Because the slidable coating film 53 contains fine particles, the slidable coating film 53 has a rough surface as shown in Fig. 10.

The inner core 52 of the guide wire 50 has the main body 52a and the distal portion 52b both made of an elastic material and integrally with each other. The diameter of the distal portion 52b is set smaller than that of the distal end of the main body 52a. Therefore the distal portion 52b has a lower rigidity than the main body 52a. The distal portion 52b may be so formed that the diameter thereof becomes gradually smaller from the distal end of the main body 52a. Thereby when a force is applied to the distal end of the distal portion 52b, the distal portion 52b is gradually bent, which allows the operability of the guide wire to be improved.

It is preferable that the inner core 52 is made of super-elastic metals or stainless steel. As the super-elastic metals, it is preferable to use a Ti-Ni alloy of 49 - 58 atomic percent of Ni, a Cu-Zn alloy of 38.5 - 41.5 wt% of Zn, a Cu-Zn-X alloy of 1 - 10 wt% of X (X = Be, Si, Sn, Al, Ga), and a Ni-Al alloy of 36 - 38 atomic percent of Al. The Ti-Ni alloy is especially favorable.

The outer diameter of the main body 52a of the inner core 52 is set favorably to the range of 0.10 to 1.00mm and more favorably to 0.15 to 0.40mm. The length of the main body 52a thereof is set favorably to the range of 1000 to 4000mm and more favorably to the range of 1500 to 3000mm. The buckling strength (yield stress at load-applied time) of the main body 52a thereof is set favorably to the range of 30 to 100 Kg/mm² (22 degree C) and more favorably to the range of 40 to 55 Kg/mm². The restoring stress (yield stress at load-removed time) of the main body 52a thereof is set favorably to the range of 20 to 80 Kg/mm² (22 degree C) and more favorably to the range of 30 to 35 Kg/mm².

The outer diameter of the distal portion 52b of the inner core 52 is set favorably to the range of 0.03 to 0.15mm and more favorably to the range of 0.05 to 0.10mm. The length of the distal portion 52b thereof is set favorably to the range of 10 to 300mm and more favorably to the range of 50 to 150mm. The bending load of the distal portion 52b thereof is set favorably to the range of 0.1 to 10g and more favorably to the range of 0.3 to 6.0g. The restoring stress of the distal portion 52b thereof is set favorably to the range of 0.1 to 10g and more favorably to the range of 0.3 to 6.0g.

The above-described outer diameter of the distal portion of the inner core does not necessarily have to be entirely set to the above-described range, but may be partly set to the above-described range. The restoring stress of the main body of the inner core and that of the distal portion thereof do not necessarily have to be set to an equal value, but it is preferable to devise a method of allowing the main body of the inner core and the distal portion thereof to obtain a suitable property at a proper diameter by changing the restoring stress thereof in various thermal treatment conditions. That is, it is preferable to separately thermally treat the main body of the inner core and the distal portion thereof in such a way that the main body thereof has a larger restoring stress and that the distal portion thereof is flexible. The inner core does not necessarily have to be made of one wire, but the inner core may be composed of a plurality of parallel or twisted wires so that the above-described function of the inner core, namely, the property thereof changes stepwise or successively.

In the embodiment shown in Fig. 6, the high X-ray opaque portion 54 fixed to the distal end of the inner core 52 is made of the annular metal member having high radiographic visualization. More specifically, the high X-ray opaque portion 54 is formed of a pipe-shaped metal member. As metals having the high radiographic visualization, gold, platinum, lead, silver, bismuth, and tungsten are favorable. Gold is especially favorable.

The high X-ray opaque portion 54 is fixed to the distal end of the inner core 52 by means of mechanical crimping, plating or soldered to an evaporated metal.

The outer diameter of the high X-ray opaque portion 54 is set favorably to the range of 0.20 to 0.90mm and more favorably to the range of 0.25 to 0.40mm. The inner diameter of the high X-ray opaque portion 54 is set favorably to the range of 0.04 to 0.16mm and more favorably to the range of 0.06 to 0.11mm. The length of the high X-ray opaque portion 54 is set favorably to the range of 1.00 to 10.00mm and more favorably to the range of 1.5 to 4.0mm.

The high X-ray opaque portion 54 may be composed of a coiled narrow wire made of the above-described metal having a high radiographic visualization. The narrow wire having a diameter of 0.02 to 0.10mm can be preferably used. The length of the high X-ray opaque portion 54 wound around the inner core 52 in the range from the distal end thereof is set to the range of 1.0 to 10.0mm and favorably to the range of 1.5 to 4.0mm.

It is preferable that as shown in Fig. 6, the slidable coating film 53 coating the entire inner core 52 has an almost uniform outer diameter, including the distal portion thereof. The outer diameter of the slidable coating film 53 is set almost uniformly over the whole length thereof by adjusting the thickness thereof in such a way that a stepped portion of the high X-ray opaque portion 54 formed at the distal end of the inner core 52 does not adversely affect the outer configuration of the guide wire 50.

As the slidable coating film 53, it is possible to preferably use the same slidable coating film as the slidable coating film 3 formed on the above-described gasket

The outer diameter of the slidable coating film 53 is set to the range of 0.25 to 1.04mm and favorably to the range of 0.30 to 0.64mm. The thickness of the inner core 52 disposed around the main body 52a is set to the range of 0.25 to 1.04mm and favorably to the range of 0.30 to 0.64mm.

It is preferable that the distal end of the guide wire 50 (distal end of slidable coating film 53) is curved, namely, hemispherical as shown in Fig. 6 to prevent walls of blood vessels from being damaged and improve the operability of the guide wire 50.

In the guide wire 50 of this embodiment, the entire inner core 52 is covered with the slidable coating film 53. But the mode of the guide wire 50 is not limited to this mode. A part of the inner core 52 may be covered with the slidable coating film 53. For example, only the distal portion or only the main body of the inner core 52 may be covered with the slidable coating film 53.

### • EXAMPLES

The examples of the present invention are described below.

### • Examples 1 through 16 and Comparison Examples 1 through 13

A surface active agent (nonionic surface active agent, commercial name: "Reodoll AO-15V" produced by Kao Co., Ltd.) was added to a solvent (toluene). After the carbon nanotubes were added to the solvent, the solution was stirred to prepare a liquid in which the carbon nanotubes were preferably dispersed. The solvent-based silicone rubber (commercial name: "YSR 3022" containing 30 wt% of silicone rubber component such as polyalkylsiloxane or the like produced by GE Toshiba Silicone Co., Ltd.) was added to the liquid in which the carbon nanotubes had been dispersed. Thereafter a filler (fine particle of silicone resin, powder of mica, powdery resin of polyolefin family), an adhesion-improving agent (commercial name "XC9603", containing silane coupling agent, which was produced by GE Toshiba Silicone Co., Ltd.), a catalyst (commercial name "YC6831", containing dialkyl tin compounds, which was produced by GE Toshiba Silicone Co., Ltd.) were added to the liquid. Thereby the (slidable) composition of each of the examples of the present invention and the comparison examples was prepared.

As the fine particle of the silicone resin, "Tospearl 145" ((commercial name) produced by GE Toshiba Silicone Co., Ltd.) was used. The "Tospearl 145" consisted of polymethyl silsesquioxane having an average diameter of 4.5µm and true specific gravity of 1.32 (25°C). As the powdery mica, the unswelling-type mica (commercial name: "PDM-5B" consisting of fluorine phlogopite produced by Topy Kogyo Co., Ltd., average diameter: 6µm) was used. As the powdery resin of the polyolefin family, powder of polyethylene (spherical, average diameter 5µm) was used.

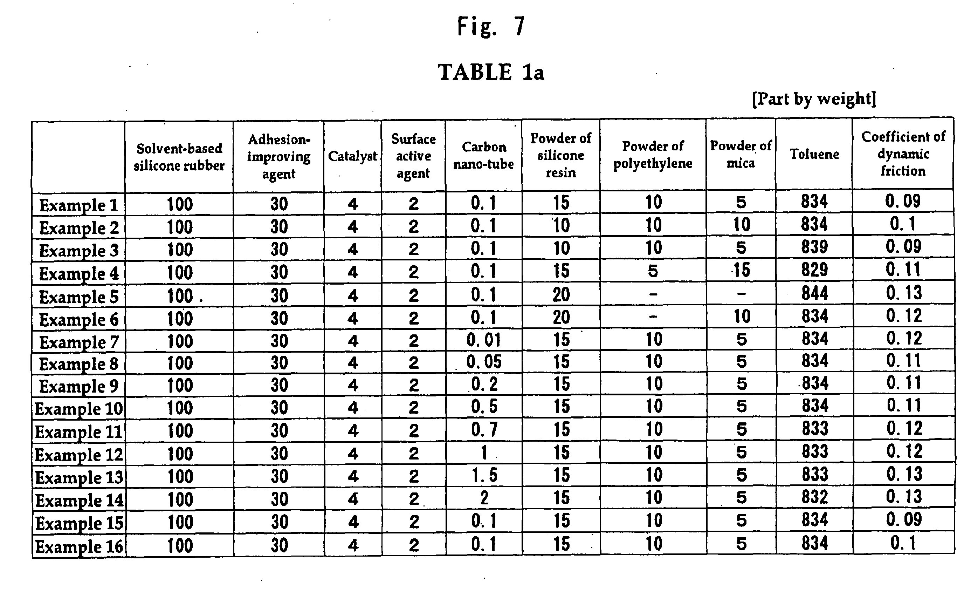

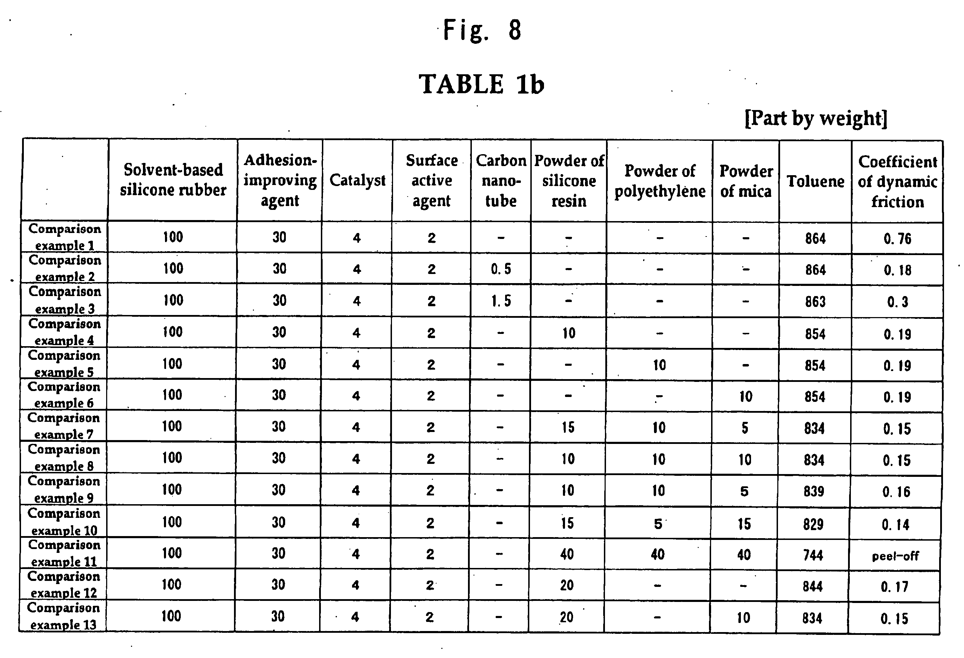

In all the examples and the comparison examples, the amount of the toluene was adjusted so that the content of the solvent-based silicone rubber contained in the composition was 10 wt%. The components of the compositions of examples and comparison examples and compounding rates thereof are as shown in table 1a and 1b of Fig. 7 and Fig. 8.

The following three kinds of carbon nanotubes were used: Type A (used in only example 15) having an average diameter of about 70nm (about *φ* 20 to 120nm) and an average length of about 0.1mm; Type B (used in only example 16) having an average diameter of about 350nm (about *φ* 200 to 500nm) and an average length of about 0.1mm; and Type C (used in all of examples and comparison examples other than 15 and 16) having an average diameter of about 150nm (about *φ* 100 to 200nm) and an average length of about 0.1mm.

### • Experiment 1

The coefficient of dynamic friction of each composition was measured in accordance with the method of JIS K 7125 by using a tribo-gear (Tribo-gear TYPE-14DR manufactured by HEIDON).

More specifically, the composition of each of the examples 1 through 16 and the comparison examples 1 through 13 was sprayed to a substrate consisting of butyl rubber (hardness: A70, Ra≒0.4µm). The components and compounding rates of each composition are as shown in table 1a and 1b. The prepared sample of each composition was brought into contact with a stainless steel terminal (*φ* 10mm, Ra≒ 0.4µm) for measuring the coefficient of friction of each composition. The coefficient of friction of each sample was measured when the sample was slid 10mm at a speed of 100mm/minute, with a load of 200g being vertically applied to the terminal. After the terminal was slid, the surface state of the coating film was visually checked. The coefficient of dynamic friction of the substrate made of butyl rubber was 1.42, when the coating agent was not applied thereto.

Fig. 9 shows the image of the coating film, made of the composition of the example 1, which was formed on the substrate of the butyl rubber. The image was obtained by using an electron microscope.

From the comparison between the composition of the examples 1, 7 through 14 and that of the comparison example 7; between the composition of the example 2 and that of the comparison example 8; between the composition of the example 3 and that of the comparison example 9; between the composition of the example 4 and that of the comparison example 10; between the composition of the example 5 and that of the comparison example 12; and between the composition of the example 6 and that of the comparison example 13, it was confirmed that the coefficient of dynamic friction decreased owing to the addition of the carbon nanotube to the rubber component (solvent-based silicone rubber). It could be also confirmed that the slidableness-improving effect can be favorably obtained when the mixing amount of the carbon nanotube for 100 parts by weight of the solvent-based silicone rubber is in the range of 0.01 to 2 parts by weight It could be confirmed from the results of the composition of the examples 1 through 6 that the compositions show a low coefficient of dynamic friction owing to the addition of the carbon nanotube to the rubber component, even though the compositions of the powder of the silicone resin, polyethylene, and mica changed to some extent

From the comparison between the composition of the example 2 and that of the comparison example 8, it was confirmed that the addition of the carbon nanotube to the rubber component caused the coefficient of dynamic friction to decrease to a higher extent than the extent of the effect which can be brought about by the addition of PTFE to the rubber component

From the experimental result of the composition of the comparison example 11, it was confirmed that when the amount of the filler added to 100 parts by weight of the solvent-based silicone rubber is not less than 100 parts by weight, peel-off (decrease of wear resistance) of the coating film occurred. From the comparison of the compositions of the example 1, 15, 16, it could be confirmed that the slidableness of the composition could be improved by the addition of the carbon nanotube to the rubber component although the diameters of the carbon nanotubes were different from one another.

### • Example 17

The core part of a gasket for use in a syringe having the configuration shown in Figs. 1 and 2 was formed by using butyl rubber. The core part was formed by press-molding a vulcanizing rubber composition composed of the butyl rubber and additives added thereto. As the configuration of the obtained core part, it had a length of 20mm, an outer diameter of 23.7mm at distal-side and proximal-side annular ribs, a length of 10mm between the center of the distal-side annular rib and the center of the proximal-side annular rib, an outer diameter of 21.5mm at a same diameter portion of the core part which extend from the distal-side annular rib to the proximal-side annular rib, a length (depth) of 8mm in the plunger-mounting concave portion having a female screw at an inner side thereof, an inner diameter of 14.5mm at the distal side of the plunger-mounting concave portion, and an inner diameter of 15mm at the rear side of the plunger-mounting concave portion.

At a room temperature and a normal pressure, the composition of the example 1 was sprayed to the core part of the gasket formed as described above. Thereafter the composition was dried at 110°C for 10 minutes. Thereby the gasket for use in the syringe was formed.

The average thickness of the slidable coating film formed on the core member was about 7µm. The ratio of the diameter of the fine particle to the average film thickness of the slidable coating film was about 0.7.

### • Comparison Example 14

A gasket for use in a syringe of the comparison example 14 was formed in a manner similar to that used in the example 17 except that the composition of the comparison example 7 was used. The average thickness of the slidable coating film formed on the core member was about 7µm.

### • Comparison Example 15

A syringe (30ml, silicone oil was applied to syringe manufactured by Terumo Kabushiki Kaisha) in which a gasket made of SEBS thermoplastic elastomer was mounted was used in the comparison example 15.

### • Comparison Example 16

Except that the composition was not applied to the core member, a gasket made of butyl rubber formed in a manner similar to that used in the example 17 was used in the comparison example 16.

### • Experiment 2

Polypropylene (produced by Nippon Polychem Co., Ltd.) was injection-molded to form outer cylinders, for use in a syringe, which had a configuration shown in Fig. 5. The cylindrical portion of the outer cylinder for use in the syringe had an inner diameter of 23.5mm and a length of 95mm. Polypropylene (produced by Nippon Polychem Co., Ltd.) was injection-molded to form plungers shown in Fig. 5.

The gaskets of the example 17 and the comparison examples 14 through 16 and the plungers were mounted on the outer cylinders to form some syringes. Silicone oil was applied to the inner surface of the outer cylinder of the comparison example 15. Except the syringe of the comparison example 5, the syringes were sterilized by autoclaving. Thereafter the following evaluations were made.

The sliding resistance value in each syringe was measured by an autograph (AG-IS manufactured by Shimazu Seisakusho Co., Ltd.). More specifically, with the distal end of each syringe and the proximal end of the plunger fixed to a fixing portion of the autograph to which an object to be measured is fixed, the plunger was moved downward 30mm at a speed of 50mm/minute to measure a maximum sliding resistance value (maximum stress [N] required in section of 0 to 30mm) and an average sliding resistance value (average sliding resistance value [N] in section of 25 to 50mm). Table 2 shows the results.

**Table 2**

| | Example 17 | Comparison Example 14 | Comparison Example 15 | Comparison Example 16 |
|---|---|---|---|---|
| Maximum sliding resistance value (N) | 5.8 | 11.5 | 20 | 93.7 |
| Average sliding resistance value (N) | 4.9 | 10.2 | 0.7 | 90.5 |

As shown in table 2, the syringe using the gasket of the example 17 had smaller maximum and average sliding resistance values than the syringes using the gaskets of the comparison examples. In addition, the syringe of the example 17 had a small difference between the maximum sliding resistance value and the average sliding resistance value. Thus there is little fear that a liquid medicine was discharged from the syringe when an operation of pressing the plunger starts, which allowed the liquid medicine to be discharged safely and easily.

### • Experiment 3

As a material to form an outer cylinder for use in a syringe, annular polypropylene (ZEONEX: registered trademark produced by Zeon Corporation) was injection-molded to form the outer cylinder for use in the syringe having a configuration shown in Fig. 5. Except that the outer cylinder for use in the syringe was used, the sliding resistance values of the gaskets for syringes of the example 17 and the comparison examples 14 through 16 were measured in a manner similar to that used in the experiment 1. Table 3 shows the results.

**Table 3**

| | Example 17 | Comparison Example 14 | Comparison Example 15 | Comparison Example 16 |
|---|---|---|---|---|
| Maximum sliding resistance value (N) | 3.6 | 4.8 | 2.1 | 23.1 |
| Average sliding resistance value (N) | 2.0 | 3.6 | 0.6 | 17.0 |

### • Example 18

A base material of a guide wire including an inner core made of Ni-Ti having an outer diameter of 0,889 mm (0.035 inches) and a length of 450mm and a polyurethane coating film formed on the surface of the inner core was prepared. After the composition of the example 1 was applied to the entire outer surface of the base material of the guide wire by using a dip coating method at a room temperature and a normal pressure, the composition was dried at 110°C for 10 minutes to obtain a guide wire of the present invention.

The average thickness of the slidable coating film formed on the surface of the inner core was about 5µm. The ratio of the diameter of the fine particle to the average film thickness of the slidable coating film was 1. Fig. 10 shows the image of the guide wire of this embodiment obtained by using an electron microscope.

### • Comparison Example 17

A guide wire of the comparison example 17 was formed in a manner similar to that used in the example 18 except that the composition of the comparison example 7 was used. The average thickness of the slidable coating film formed on the core member was about 7µm.

### • Comparison Example 18

A guide wire of the comparison example 18 was formed in a manner similar to that used in the example 18 except that reactive silicone oil was applied to the core member instead of coating the core member with the composition.

### • Comparison Example 19

A guide wire (only inner core) of the comparison example 19 was formed in a manner similar to that used in the example 18 except that the composition was not applied to the core member of the guide wire.

### • Experiment 4

The sliding resistance value of each of guide wires of the example 18 and the comparison examples 17 through 19 was measured by the autograph (Autograph AG-IS manufactured by Shimazu Seisakusho Co., Ltd.) by sliding the guide wires on the inner surface of a dilator (made of polypropylene) which is used when the guide wire is inserted into a human body.

More specifically, the dilator was fixed to a jig (jig for curving the central portion of the body thereof into a circle having a radius of 25mm). After the guide wire was inserted into the dilator, the distal end of the guide wire was fixed to the fixing portion of the autograph to which an object to be measured is fixed. In this state, the guide wire was pulled upward 100mm at a speed of 100mm/minute to measure a maximum sliding resistance value (maximum stress [gf] required in section of 0 to 100mm) and an average sliding resistance value (average sliding resistance value [gf] in section of 5 to 100mm). Table 4 shows the results.

**Table 4**

| | Example 18 | Comparison Example 17 | Comparison Example 18 | Comparison Example 19 |
|---|---|---|---|---|
| Maximum sliding resistance value(gf) | 5.8 | 10.2 | 19.9 | 50.9 |
| Average sliding resistance value (gf) | 5.0 | 9.0 | 17.3 | 39.4 |

It was confirmed that the guide wire (guide wire coated with composition of example 1) had slidableness improved over that of the comparison example 17 (guide wire coated with composition of comparison example 7), the comparison example 18 (guide wire having reactive silicone oil applied to inner core thereof), and the comparison example 19 (non-coated guide wire). It was confirmed that the guide wire of the example 18 had improved slidableness owing to the addition of the carbon nanotube to the rubber component

The medical appliance-coating composition of the present invention contains the solvent-based silicone rubber as its main component and in addition the carbon nanotubes and the fine particles of the silicone resin.

Therefore the coating film made of the medical appliance-coating composition has a high degree of slidableness and strength and is capable of imparting a sufficiently high degree of slidableness to the medical appliance.

The medical appliance of the present invention moves in contact with the inner surface of the medical member or the inner surface of the lumen and has the slidable coating film formed at the portion thereof which contacts the medical member or the lumen. The slidable coating film is composed of the silicone rubber and contains the carbon nanotubes and the fine particles of the silicone resin.

Therefore the slidable coating film formed on the medical appliance has a high degree of slidableness and strength. Thus the medical appliance keeps a sufficient degree of slidableness for a long time.

## Claims

1. A medical appliance-coating composition for imparting slidableness to said medical appliance which contains a solvent-based silicone rubber, carbon nanotubes, and fine particles of silicone resin, having an average diameter of as to 30 µm.

2. A medical appliance-coating composition according to claim t wherein said solvent-based silicone rubber is silicone resin having polysiloxane as a basic structure thereof; and a content of said silicone resin having said polysiloxane as said basic structure thereof is 10 to 70 wt% in said medical appliance-coating composition.

3. A medical appliance-coating composition according to claim 1 or 2, wherein said carbon nanotubes have an average diameter set to less than 800nm and an average fiber length set to less than 2mm and are contained at 0.01 to 2 wt% in said medical appliance-coating composition.

4. A medical appliance-coating composition according to any one of claims 1 through 3, wherein said fine particles of said silicone resin having an average diameter set to 0.5 to 30µm are contained at 0.2 to 50 wt% in said medical appliance-coating composition.

5. A medical appliance-coating composition according to any one of claims 1 through 4, wherein said medical appliance-coating composition contains a surface active agent.

6. A medical appliance-coating composition according to any one of claims 1 through 4, wherein said medical appliance-coating composition contains a nonionic surface active agent.

7. A medical appliance-coating composition according to any one of claims 1 through 6, wherein said medical appliance-coating composition contains powder of polyolefin resin or/ and powder of mica.

8. A medical appliance-coating composition according to claim 7, wherein said medical appliance-coating composition contains 0.1 to 50 wt% of said powder of said polyolefin resin or said powder of said mica.

9. A medical appliance-coating composition according to any one of claims 1 through 8, wherein said medical appliance-coating composition contains a silane coupling agent.

10. A medical appliance, having a slidable coating film formed thereon, which moves in contact with an inner surface of a medical member or an inner surface of a body lumen,
wherein said slidable coating film is disposed at a portion, of said medical appliance, which contacts said medical member or said body lumen; and said slidable coating film is comprised of a composition according to claim 1.

11. A medical appliance according to claim 10, wherein said silicone rubber has polysiloxane as a basic structure thereof.

12. A medical appliance according to claim 10 or 11, wherein said carbon nanotubes have diameters set to less than 800nm and an average fiber length set to less than 2mm and are contained at 0.01 to 3 wt% in said slidable coating film.

13. A medical appliance according to any one of claims 10 through 12, wherein said fine particles of said silicone resin have an average diameter set to 1 to 30µm and are contained at 3 to 60 wt% in said slidable coating film.

14. A medical appliance according to any one of claims 10 through 13, wherein said slidable coating film contains powder of polyolefin resin or/and powder of mica.

15. A medical appliance according to any one of claims 10 through 14, wherein said slidable coating film has a rough surface attributed to presence of said fine particle of said silicone resin and/or said carbon nanotubes contained therein.

16. A medical appliance according to any one of claims 10 through 15, wherein said medical member is an outer cylinder for use in a syringe; and said medical appliance is a gasket for use in said syringe.

17. A medical appliance according to any one of claims 10 through 15, wherein said medical appliance is a guide wire or a catheter for inserting said body lumen.

## Patentansprüche

1. Beschichtungszusammensetzung für medizinische Geräte, um dem medizinischen Gerät Gleitfähigkeit zu verleihen, wobei die Zusammensetzung einen lösungsmittelhaltigen Silikonkautschuk, Kohlenstoffnanoröhrchen und feine Partikel aus Silikonharz mit einem durchschnittlichen Durchmesser von 0,5 bis 30 µm enthält.

2. Beschichtungszusammensetzung für medizinische Geräte nach Anspruch 1, wobei der lösungsmittelhaltige Silikonkautschuk Silikonharz ist, das Polysiloxan als Grundstruktur hat; und wobei der Gehalt an Silikonharz mit dem Polysiloxan als Grundstruktur 10 bis 70 Gew.-% in der Beschichtungszusammensetzung für medizinische Geräte beträgt.

3. Beschichtungszusammensetzung für medizinische Geräte nach Anspruch 1 oder 2, wobei die Kohlenstoffnanoröhrchen einen auf kleiner als 800 nm eingestellten durchschnittlichen Durchmesser und eine auf kleiner als 2 mm eingestellte durchschnittliche Faserlänge haben und in einer Menge von 0,01 bis 2 Gew.-% in der Beschichtungszusammensetzung für medizinische Geräte enthalten sind.

4. Beschichtungszusammensetzung für medizinische Geräte nach einem der Ansprüche 1 bis 3, wobei die feinen Partikel aus dem Silikonharz mit einem auf 0,5 bis 30 µm eingestellten durchschnittlichen Durchmesser in einer Menge von 0,2 bis 50 Gew.-% in der Beschichtungszusammensetzung für medizinische Geräte enthalten sind.

5. Beschichtungszusammensetzung für medizinische Geräte nach einem der Ansprüche 1 bis 4, wobei die Beschichtungszusammensetzung für medizinische Geräte einen grenzflächenaktiven Stoff enthält.

6. Beschichtungszusammensetzung für medizinische Geräte nach einem der Ansprüche 1 bis 4, wobei die Beschichtungszusammensetzung für medizinische Geräte einen nichtionogenen grenzflächenaktiven Stoff enthält.

7. Beschichtungszusammensetzung für medizinische Geräte nach einem der Ansprüche 1 bis 6, wobei die Beschichtungszusammensetzung für medizinische Geräte Polyolefinharzpulver und/oder Glimmerpulver enthält.

8. Beschichtungszusammensetzung für medizinische Geräte nach Anspruch 7, wobei die Beschichtungszusammensetzung für medizinische Geräte 0,1 bis 50 Gew.-% des Polyolefinharzpulvers oder des Glimmerpulvers enthält.

9. Beschichtungszusammensetzung für medizinische Geräte nach einem der Ansprüche 1 bis 8, wobei die Beschichtungszusammensetzung für medizinische Geräte einen Silanhaftvermittler enthält.

10. Medizinisches Gerät mit einem darauf ausgebildeten gleitfähigen Beschichtungsfilm, der sich in Kontakt mit einer Innenfläche eines medizinischen Elements oder einer Innenfläche eines Körperlumens bewegt,
wobei der gleitfähige Beschichtungsfilm auf einem Abschnitt des medizinischen Geräts angeordnet ist, der das medizinische Element oder das Körperlumen berührt; und wobei der gleitfähige Beschichtungsfilm aus einer Zusammensetzung nach Anspruch 1 besteht.

11. Medizinisches Gerät nach Anspruch 10, wobei der Silikonkautschuk Polysiloxan als Grundstruktur hat.

12. Medizinisches Gerät nach Anspruch 10 oder 11, wobei die Kohlenstoffnanoröhrchen einen auf kleiner als 800 nm eingestellten Durchmesser und eine auf kleiner als 2 mm eingestellte durchschnittliche Faserlänge haben und in einer Menge von 0,01 bis 3 Gew.-% in dem gleitfähigen Beschichtungsfilm enthalten sind.

13. Medizinisches Gerät nach einem der Ansprüche 10 bis 12, wobei die feinen Partikel aus dem Silikonharz einen auf 1 bis 30 µm eingestellten durchschnittlichen Durchmesser haben und in einer Menge von 3 bis 60 Gew.-% in dem gleitfähigen Beschichtungsfilm enthalten sind.

14. Medizinisches Gerät nach einem der Ansprüche 10 bis 13, wobei der gleitfähige Beschichtungsfilm Polyolefinharzpulver und/oder Glimmerpulver enthält.

15. Medizinisches Gerät nach einem der Ansprüche 10 bis 14, wobei der gleitfähige Beschichtungsfilm eine rauhe Oberfläche besitzt, die auf die Gegenwart der feinen Partikel aus dem Silikonharz und/oder die darin enthaltenen Kohlenstoffnanoröhrchen zurückzuführen ist.

16. Medizinisches Gerät nach einem der Ansprüche 10 bis 15, wobei das medizinische Element ein äußerer Zylinder zur Verwendung in einer Spritze ist und das medizinische Gerät eine Dichtung zur Verwendung in der Spritze ist.

17. Medizinisches Gerät nach einem der Ansprüche 10 bis 15, wobei das medizinische Gerät ein Führungsdraht oder ein Katheter zum Einführen in das Körperlumen ist.

## Revendications

1. Composition de revêtement pour dispositif médical, conçue pour conférer à ce dispositif médical une certaine aptitude à glisser, qui contient un caoutchouc de silicone en base solvant, des nanotubes de carbone, et de fines particules de résine de silicone dont le diamètre moyen vaut de 0,5 à 30 µm.

2. Composition de revêtement pour dispositif médical, conforme à la revendication 1, dans laquelle ledit caoutchouc de silicone en base solvant est une résine de silicone dont la structure de base est celle d'un polysiloxane, et dans laquelle composition de revêtement pour dispositif médical la proportion pondérale de cette résine de silicone à structure de base de type polysiloxane vaut de 10 à 70 %.

3. Composition de revêtement pour dispositif médical, conforme à la revendication 1 ou 2, dans laquelle lesdits nanotubes de carbone présentent un diamètre moyen de moins de 800 nm et une longueur de fibre moyenne de moins de 2 mm, et dans laquelle composition de revêtement pour dispositif médical ces nanotubes de carbone se trouvent présents en une proportion pondérale de 0,01 à 2 %.

4. Composition de revêtement pour dispositif médical, conforme à l'une des revendications 1 à 3, dans laquelle lesdites fines particules de résine de silicone de 0,5 à 30 µm de diamètre moyen se trouvent présentes en une proportion pondérale de 0,2 à 50 %.

5. Composition de revêtement pour dispositif médical, conforme à l'une des revendications 1 à 4, laquelle composition de revêtement pour dispositif médical contient un agent tensioactif.

6. Composition de revêtement pour dispositif médical, conforme à l'une des revendications 1 à 4, laquelle composition de revêtement pour dispositif médical contient un agent tensioactif non-ionique.

7. Composition de revêtement pour dispositif médical, conforme à l'une des revendications 1 à 6, laquelle composition de revêtement pour dispositif médical contient une résine de type polyoléfine en poudre et/ou du mica en poudre.

8. Composition de revêtement pour dispositif médical, conforme à la revendication 7, laquelle composition de revêtement pour dispositif médical contient de 0,1 à 50 % en poids de ladite résine de type polyoléfine en poudre ou dudit mica en poudre.

9. Composition de revêtement pour dispositif médical, conforme à l'une des revendications 1 à 8, laquelle composition de revêtement pour dispositif médical contient un agent de couplage de type silane.

10. Dispositif médical, sur lequel est formé un film de revêtement glissant et qu'on déplace en le laissant en contact avec une surface interne d'un ustensile médical ou avec la surface interne d'un vaisseau ou d'un organe creux du corps, lequel film de revêtement glissant est placé sur une partie dudit dispositif médical qui entre en contact avec ledit ustensile médical ou avec ledit vaisseau ou organe creux du corps, et lequel film de revêtement glissant est fait d'une composition conforme à la revendication 1.

11. Dispositif médical conforme à la revendication 10, dans lequel ledit caoutchouc de silicone présente une structure de base qui est celle d'un polysiloxane.

12. Dispositif médical conforme à la revendication 10 ou 11, dans lequel lesdits nanotubes de carbone ont moins de 800 nm de diamètre et présentent une longueur de fibre moyenne de moins de 2 mm, et se trouvent présents en une proportion pondérale de 0,01 à 3 % dans ledit film de revêtement glissant.

13. Dispositif médical conforme à l'une des revendications 10 à 12, dans lequel lesdites fines particules de résine de silicone ont de 1 à 30 µm de diamètre moyen et se trouvent présentes en une proportion pondérale de 3 à 60 % dans ledit film de revêtement glissant.

14. Dispositif médical conforme à l'une des revendications 10 à 13, dans lequel ledit film de revêtement glissant contient une résine de type polyoléfine en poudre et/ou du mica en poudre.

15. Dispositif médical conforme à l'une des revendications 10 à 14, dans lequel ledit film de revêtement glissant présente une surface rugueuse qui est due à la présence desdites fines particules de résine de silicone et/ou desdits nanotubes de carbone qu'il contient.

16. Dispositif médical conforme à l'une des revendications 10 à 15, ledit ustensile médical étant le corps d'une seringue et ledit dispositif médical étant un joint d'étanchéité utilisable dans ladite seringue.

17. Dispositif médical conforme à l'une des revendications 10 à 15, lequel dispositif médical est un fil de guidage ou un cathéter à insérer dans un vaisseau ou un organe creux du corps.
